# EUROPEAN PATENT SPECIFICATION

(11) **EP 3 859 624 B1**
(45) Date of publication and mention of the grant of the patent: **04.03.2026**
(21) Application number: 18934616.6
(22) Date of filing: 26.09.2018
(51) Int. Cl.: G06Q 10/08, G06Q 10/0832, G06Q 30/0645, G16H 40/20

(54) **RECEIVED ORDER MANAGEMENT SYSTEM, RECEIVED ORDER MANAGEMENT METHOD, AND PROGRAM**
VERWALTUNGSSYSTEM FÜR EMPFANGENE AUFTRÄGE, VERWALTUNGSVERFAHREN FÜR EMPFANGENE AUFTRÄGE UND PROGRAMM
SYSTÈME DE GESTION DE COMMANDE REÇUE, PROCÉDÉ DE GESTION DE COMMANDE REÇUE ET PROGRAMME

(43) Date of publication of application: 04.08.2021
(73) Proprietor: Medipal Holdings Corporation, Tokyo 104-8461 (JP)
(72) Inventor: NAGASAWA, Kazunori, Tokyo 104-8461 (JP)
(74) Representative: Stöckeler, Ferdinand
(86) International application number: PCT/JP2018/035605
(87) International publication number: WO 2020/065751

(56) References cited:
- WO-A1-2018/115833
- WO-A2-2004/104768
- JP-A- 2002 123 594
- JP-A- 2004 164 548
- JP-A- 2009 110 254
- JP-A- 2017 182 599
- JP-A- 2018 136 631
- US-A1- 2008 104 976
- US-A1- 2014 258 165
- US-A1- 2018 055 042

## Description

### Field

The present invention relates to a received-order management system, a received-order management method, and a program suitable for calculating a rental fee of an optimum container capable of ensuring the quality of an article.

### Background

Conventionally, a rent collection management system in which a returnable container is easily rented to a user of the returnable container such as an individual producer including small to medium-sized farmers, and charging and receipting operations of a rental fee thereof are easily performed has been known.

As an example of such a conventional rent collection management system, Patent Literature 1 has been known.

In Patent Literature 1, in order to easily rent a returnable container to a user of the returnable container such as an individual producer including small to medium-sized farmers, and perform charging and receipting operations of a rental fee, such a technique is disclosed that storage of the reusable returnable containers to be used for distribution of articles is commissioned from a lender to an operation center or the like, the operation center rents the container to article producers, and the producer returns the container to the operation center at the time of selling the articles to the operation center.

More specifically, the technique includes an input unit/reception unit of data including at least the number of returned containers and a display of a source to be returned when a producer sells articles in the returnable container to the operation center and returns the container to the operation center, a memory unit, a calculation unit that calculates a rental fee corresponding to an amount obtained by multiplying the number of the returned container in the data by a rental unit price, and an output instruction unit that issues an output instruction in order to pay the amount of the rental fee from the producer to the lender, or to make a provisional payment from the producer to the operation center. The technique has an advantage such that quality management of articles can be reliably performed and billing and return management of the returnable container can be performed simultaneously, by calculating the rental fee corresponding to the amount obtained by multiplying the number of the returned container by the rental unit price.

According to Patent Literature 1, there are such effects that the container rental to a product distribution system is further promoted, and reduction of wastes and streamlining of logistics can be realized, as compared to distribution using a conventional returnable container (such as a cardboard box) assumed to be disposed.

Patent Literature 2 discloses a portable, insulated capsules for cryopreservation of biological materials. According to Patent Literature 2, it is possible to maintain constant very low temperature and more particularly, pertains to refrigeration and storage apparatus for cryopreservation of biological materials.

### Citation List

### Patent Literature

Patent Literature 1: Japanese Patent Application Laid-open No. 2005-135155
Patent Literature 2: Unites State Patent Application Publication No. 2018/0055042

### Summary

### Technical Problem

However, in Patent Literature 1, since its main purpose is to make it easy to rent a returnable container prepared beforehand to a user, and perform charging and receipting operations of the rental fee, an optimum returnable container according to the type of articles cannot be selected. Further, in Patent Literature 2, it is not possible to selectively activate the sensors to be used according to the type of articles.

For example, when it is necessary to refrigerate articles to be transported in a low temperature state of about -170°C, a user at a delivery destination needs to prepare an expensive low-temperature storage container, which imposes a burden of large expenses on the user.

Therefore, a service form in which a storage container is rented only for a period in which delivered articles need to be stored, without requiring the user at the delivery destination to perform large investment associated with the low temperature storage container, has been desired.

One embodiment of the present invention has been achieved in view of the above circumstances, and an object of the present invention is to make it possible to select an optimum container capable of ensuring the quality of an article from a plurality of containers according to the type of the article, and to calculate a rental unit price for one container to be commonly used at the time of delivery of the article and at the time of storing the article at a delivery destination and calculate a rental fee of the container.

### Solution to Problem

In order to solve the above problem, the present application provides a received-order management system according to claim 1.

### Advantageous Effects of Invention

According to the present invention, a rental fee of one container to be used at the time of delivery of an article and at the time of storing the article at the delivery destination can be calculated according to the type of the article.

Further, a rental fee of a container that can be used at the time of delivery of an article while ensuring the quality thereof and at the time of storing the article at the delivery destination can be calculated.

### Brief Description of Drawings

[FIG. 1] FIG. 1 is a block diagram illustrating a configuration of a received-order management system according to a first embodiment of the present invention.
[FIG. 2] FIG. 2 is a diagram illustrating a vehicle on which a delivery unit according to the first embodiment of the present invention is mounted.
[FIG. 3] FIG. 3 is a diagram illustrating a hardware configuration of the delivery unit according to the first embodiment of the present invention.
[FIG. 4] FIG. 4 is a functional block diagram of a received-order management server according to the first embodiment of the present invention.
[FIG. 5] FIG. 5 is a sequence diagram of calendar screen generation according to the first embodiment of the present invention.
[FIG. 6] FIG. 6 is a diagram illustrating a personal calendar screen in a state of including a use date by a client terminal according to the first embodiment of the present invention.
[FIG. 7] FIG. 7 is an ER diagram illustrating an association of files to be processed by the received-order management server according to the first embodiment of the present invention.
[FIG. 8] FIG. 8 is a work sequence diagram illustrating a container rental procedure according to the first embodiment of the present invention.
[FIG. 9] FIG. 9 is a sequence diagram illustrating a replenishment flow to a rented container according to the first embodiment of the present invention.
[FIG. 10] FIG. 10 is a flowchart of collection determination according to the first embodiment of the present invention.
[FIG. 11] FIG. 11 is a diagram illustrating a configuration of rental information in the flowchart of collection determination according to the first embodiment of the present invention.
[FIG. 12] FIG. 12 is a flowchart of billing calculation according to the first embodiment of the present invention.
[FIGS. 13] FIG. 13(a) is a diagram illustrating a configuration of rental information, FIG. 13(b) is a diagram illustrating unit information, and FIG. 13(c) is a diagram illustrating unit configuration information, which are referred to in the flowchart of billing calculation according to the first embodiment of the present invention.
[FIG. 14] FIG. 14 is a diagram illustrating a total amount for each unit type according to the first embodiment of the present invention.
[FIG. 15] FIG. 15 is a diagram illustrating a monitoring/status display screen displayed on a PC in the delivery unit according to the first embodiment of the present invention.
[FIG. 16] FIG. 16 is a diagram illustrating an operation screen to be used at the time of finishing monitoring of the delivery unit according to the first embodiment of the present invention.
[FIG. 17] FIG. 17 is a perspective view illustrating a modification of the delivery unit according to a second embodiment of the present invention.
[FIG. 18] FIG. 18 is a diagram illustrating a hardware configuration of the modification of the delivery unit according to the second embodiment of the present invention.

### Description of Embodiments

The present invention will be described below with embodiments illustrated in the drawings.

The present invention has a configuration described below, in order to calculate a rental fee of one container to be used at the time of delivery of an article and at the time of storing the article at a delivery destination according to the type of the article, or to calculate a rental fee of a container that can be used at the time of delivery of an article while ensuring the quality thereof and at the time of storing the article at the delivery destination.

That is, a received-order management system according to the present invention includes a received-order management server that generates received-order data and a delivery unit for delivering a container that accommodates therein an article, and marages delivery of the delivery unit and collection of the delivery unit by connecting the received-order management server and the delivery unit with each other via a communication network. The delivery unit can mount therein a specific type of container from plural kinds of containers prepared according to the type of the article. The received-order management server includes a schedule calculation unit that calculates delivery date data associated with delivery of the article and collection date data associated with collection of the delivery unit, based on an article code and use date data received from a user terminal on a user side, a selection unit that selects a container of a type corresponding to the article from plural kinds of containers based on the article code, and a billing-amount calculation unit that calculates a billing amount by multiplying a rental period calculated based on a delivery completion notification and a collection completion notification received from the delivery unit by a unit price of the selected container.

With the above configuration, the rental fee of one container to be used at the time of delivery of an article and at the time of storing the article at the delivery destination can be calculated according to the type of the article.

Further, the rental fee of a container that can be used at the time of delivery of an article while ensuring the quality thereof and at the time of storing the article at the delivery destination can be calculated.

Characteristics of the present invention described above are explained in detail with reference to the drawings mentioned below. Note that, unless otherwise specified, constituent elements, types, combinations, shapes, and relative arrangements thereof described in the following embodiments are not intended to limit the scope of the present invention solely thereto and are only explanatory examples.

Characteristics of the present invention described above are explained below in detail with reference to the drawings.

### <First embodiment>

### <Received-order management system>

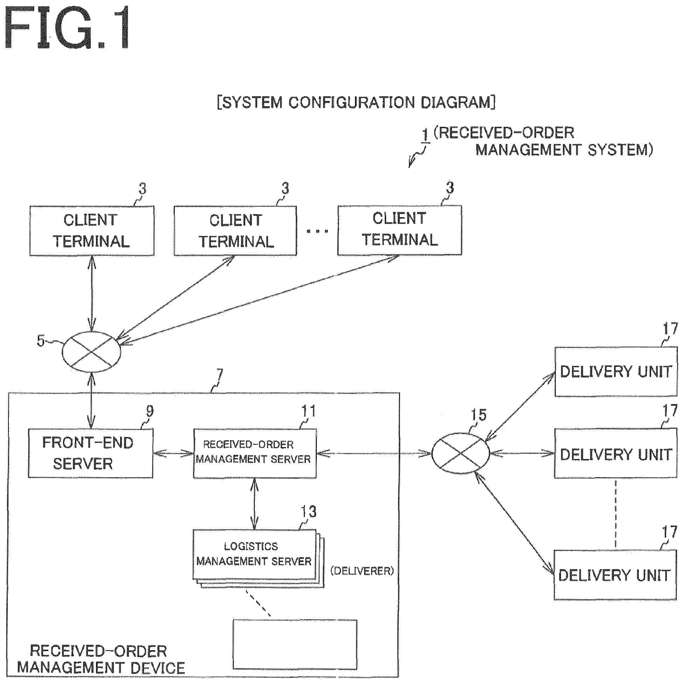

FIG. 1 is a block diagram illustrating a configuration of a received-order management system according to a first embodiment of the present invention.

In the following descriptions, like constituent elements are explained while being denoted by like reference signs.

A received-order management system 1 is configured to include a client terminal 3, communication networks 5 and 15, a received-order management device 7, a delivery unit 17, and a logistics management server (a deliverer) 13.

The received-order management device 7 is configured to include a front-end server 9 and a received-order management server 11.

In the present embodiment, each of the client terminal 3, the delivery unit 17, and the logistics management server 13 is provided in plural. However, each of the units may be provided in singular. Further, the communication network is divided into 5 and 15. However, these networks may be configured by the same network.

The front-end server 9 has a function of receiving data from the client terminal 3 via the network 5 to provide direct access service to the client terminals 3 and change a display format.

The client terminal 3 is a terminal operable by a delivery destination.

The received-order management server 11 receives data of the delivery unit 17 via the communication network 15 to manage the state of each delivery unit 17.

The received-order management server 11 has therein a ROM (Read Only Memory), a RAM (Random Access Memory), a CPU (Central Processing Unit), and an HDD (Hard Disk Drive). The received-order management server 11 reads an operating system OS from the HDD and develops the OS on the RAM to activate the OS, and reads programs (programs indicated by various flowcharts described later) from the HDD to perform various types of processing, under control of the OS.

The logistics management server 13 is arranged in each warehouse that stores each article or in each warehouse that stores a plurality of articles, and executes control for shipping a corresponding article to a client upon reception of a request from the received-order management server 11.

In the delivery unit 17, a GPS receiver 37 receives each GPS signal from GPS satellites. The delivery unit 17 then calculates location data based on each GPS signal, adds a unique device code thereto, and transmits the location data to the received-order management server 11 via the communication network 15.

It suffices that the unique device code provided to the GPS receiver 37 is, for example, a MAC address (Media Access Control address) of the GPS receiver 37 or a unique management number set beforehand.

### <Vehicle>

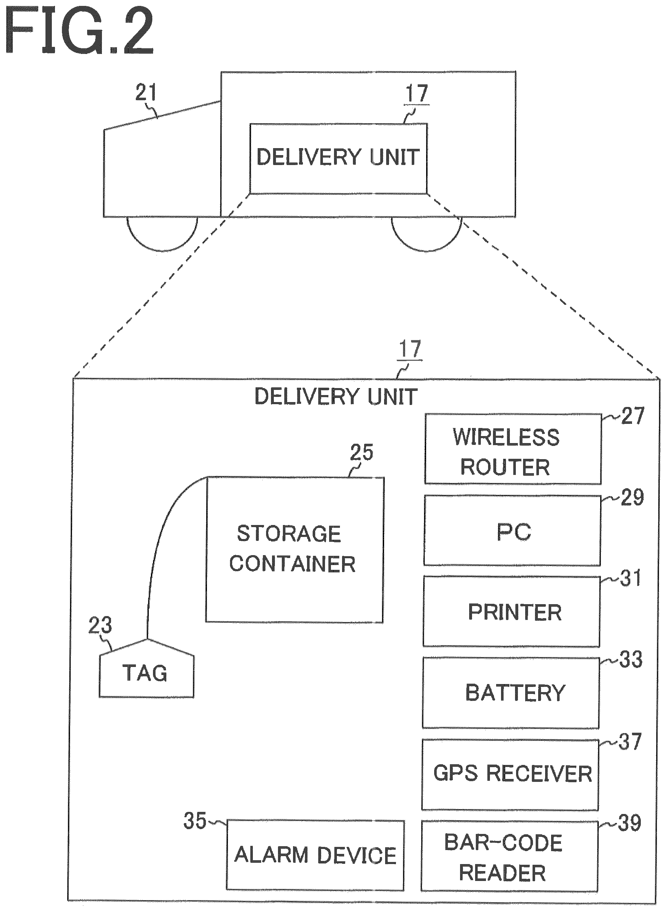

FIG. 2 is a diagram illustrating a vehicle on which the delivery unit according to the first embodiment of the present invention is mounted.

A vehicle 21 is mounted with the delivery unit 17 on a loading platform thereof. Further, the delivery unit 17 includes a storage container 25, a wireless router 27, a personal computer (hereinafter, PC) 29, a printer 31, a battery 33, an alarm device 35, the GPS receiver 37, and a bar-code reader 39.

A plurality of sensors described later are accommodated in or attached to the storage container 25, and a part of a tag string fastened to each article accommodated in the storage container 25 is pulled outside, and a tag 23 is fastened at the end thereof.

### <Delivery unit>

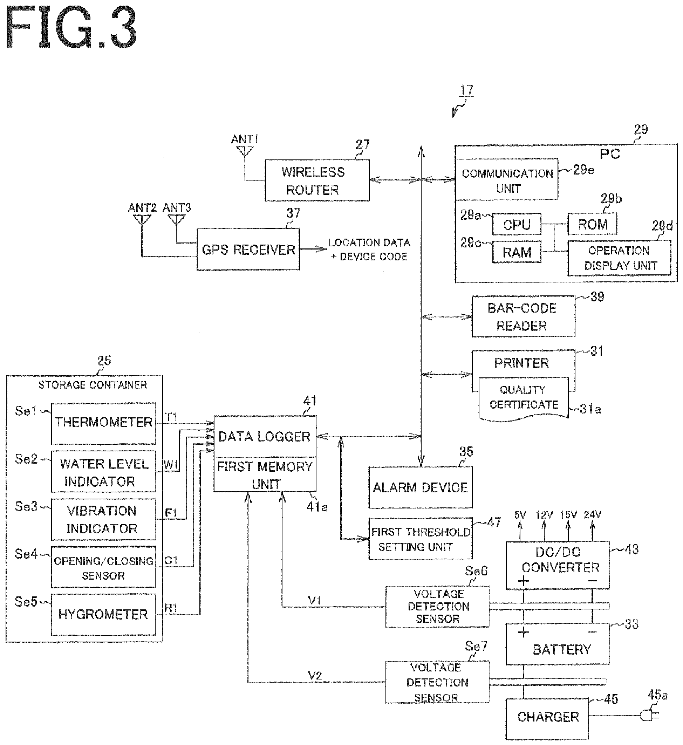

FIG. 3 is a diagram illustrating a hardware configuration of the delivery unit according to the first embodiment of the present invention.

The delivery unit 17 includes the storage container 25, the wireless router 27, the PC 29, the printer 31, the battery 33, the alarm device 35, the GPS receiver 37, the bar-code reader 39, a data logger 41, a DC/DC converter 43, a charger 45, and a first threshold setting unit 47. The delivery unit 17 includes a plurality of devices (various sensors Se1 to Se5) described later, and a used state/unused state of each device can be set from outside.

The delivery unit 17 can mount therein a specific type of container from plural kinds of containers prepared according to the type of the article.

Here, the type of the article means that the articles are different from each other according to the property, application, stored temperature, and stored humidity of the article. Further, the type of the container means that the containers have a different stored temperature and a stored humidity from each other corresponding to the type of the article.

The storage container 25 can accommodate therein a plurality of articles, and includes the various sensors Se1 to Se5 therein or attached to the outside thereof.

The thermometer Se1 is provided in the storage container 25 to measure an ambient temperature in the storage container 25, and outputs temperature data (T1) to the data logger 41.

The liquid level indicator Se2 is provided in the storage container 25 to measure a liquid level of a liquefied cooling medium, for example, liquid nitrogen and outputs liquid level data (W1) to the data logger 41.

The vibration indicator Se3 is provided outside the storage container 25 to measure vibrations applied to the storage container 25, and outputs vibration data (F1) to the data logger 41.

The opening/closing sensor Se4 is provided in the storage container 25 to detect whether a lid of the storage container 25 is in an opened state or a closed state and outputs opening/closing data (C1) to the data logger 41.

The hygrometer Se5 is provided in the storage container 25 to measure the ambient humidity in the storage container 25
, and outputs humidity data (R1) to the data logger 41.

A voltage detection sensor Se6 measures a voltage of the DC power supplied from the battery 33 to the DC/DC converter 43, and outputs voltage data (V1) to the data logger 41.

A voltage detection sensor Se7 measures a voltage of the DC power supplied from the charger 45 to the battery 33, and outputs voltage data (V2) to the data logger 41.

The wireless router 27 accesses a plurality of 3G/4G lines arranged on the road where the vehicle 21 runs, and is connected to the PC 29 to connect the PC 29 and the received-order management server 11 with each other.

The PC 29 includes a CPU 29a, a ROM 29b, a RAM 29c, an operation display unit 29d, and a communication unit 29e.

The CPU 29a controls the entire operation of the delivery unit 17 by using the RAM 29c as a work memory, according to a program memorized beforehand in the ROM 29b.

The ROM 29b is a read-only non-volatile memory medium, and stores therein firmware and various kinds of data.

The RAM 29c is a volatile memory medium capable of high-speed read and write of information and can be used as a work memory.

The operation display unit 29d includes a screen and key buttons for displaying a menu for performing various kinds of setting and mode selection, and receives various kinds of operation requests from a user. For example, a used state/unused state of plural devices (the various kinds of the sensors Se1 to Se5) described later can be set by the operation display unit 29d.

The communication unit 29e has a USB interface, and transmits and receives data to and from the received-order management server 11 via the wireless router 27 and the communication network 15.

The printer 31 prints a quality certificate 31a on a recording medium such as a sheet according to a printing job received from the PC 29.

When the charger 45 is connected to each electrode (ON), the battery 33 is charged with the DC power supplied from the charger 45, and when connection between the charger 45 and the respective electrodes is released (OFF), the battery 33 discharges the DC power and supplies the DC power to the DC/DC converter 43.

When the data exceeds the threshold, the alarm device 35 is activated to activate an alarm, and notifies this matter to a person in charge by blinking the warning light.

The GPS receiver 37 receives a radio signal from a plurality of GPS satellites via an antenna ANT 2 to calculate location information of the delivery unit 17, and transmits the calculated location information to the received-order management server 11 via an antenna ANT 3 and/or the network 15. In FIG. 3, the GPS receiver 37 is not connected to the PC 29. However, the GPS receiver 37 may be connected to the PC 29 to output the location information calculated by the GPS receiver 37 to the PC 29.

The bar-code reader 39 reads a bar-code and outputs data to the PC 29.

The data logger 41 collects measurement data measured by each sensor, stores various kinds of data therein, and outputs the stored various kinds of data to the PC 29 according to readout of the PC 29. The data logger 41 includes a first memory unit 41a that memorizes therein temperature data measured by the thermometer Se1 or data measured by other sensors in time series.

The DC/DC converter 43 converts the DC power supply supplied from the battery 33 to DC power of, for example, four kinds of voltage levels (5V, 12V, 15V, and 24V) and supplies the DC power to each unit in the delivery unit 17.

The charger 45 is connected to, for example, an AC power supply provided in the vehicle 21 or an AC power supply provided in a delivery center via a plug 45a, as needed, to convert AC power supplied from the AC power supply to DC power, and connects the DC power to each electrode of the battery 33 to charge the battery 33.

The first threshold setting unit 47 sets a threshold of each of the temperature data, the liquid level data, the vibration data, the humidity data, and the opening/closing number data.

### <Functional block of received-order management server>

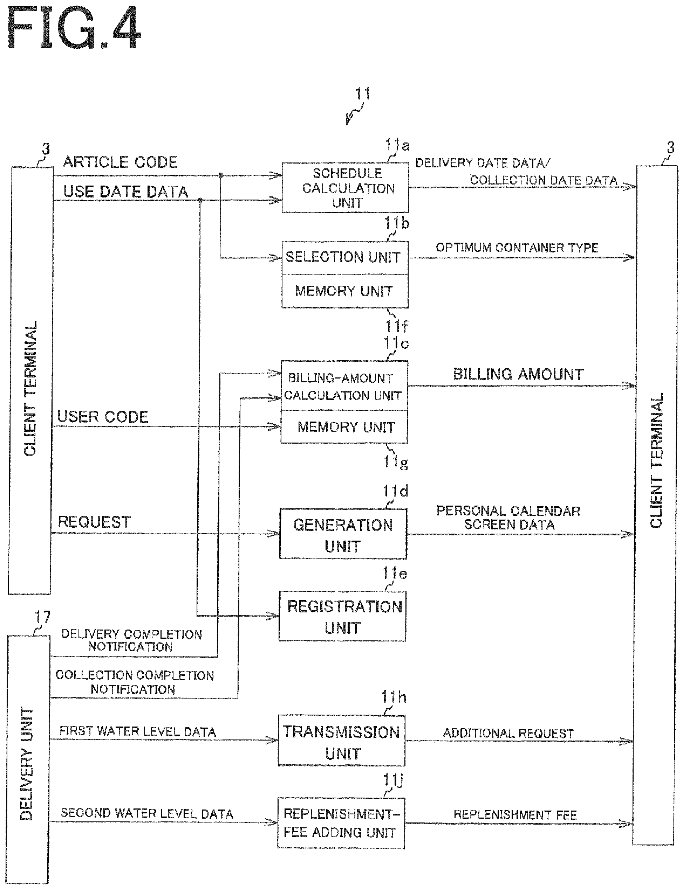

FIG. 4 is a functional block diagram of the received-order management server according to the first embodiment of the present invention.

A schedule calculation unit 11a calculates delivery date data associated with delivery of the article and collection date data associated with collection of the delivery unit, based on the article code and the use date data received from the client terminal 3 of the user side.

A selection unit 11b selects a container of a type corresponding to the article from plural kinds of containers based on the article code.

A billing-amount calculation unit 11c calculates a billing amount by multiplying a rental period calculated based on a delivery completion notification representing that the delivery unit 17 has arrived at a user side and a collection completion notification representing that a collection operation has been completed by a collection operation to be performed on a collection date calculated at the time of placing an order by a unit price of the selected container.

A generation unit 11d generates personal calendar screen data so as to include a region for specifying an article code, a region for specifying personal identification data, a region for specifying delivery destination data of the article, and a calendar region for an individual (a patient) that includes a plurality of date regions arranged per month and can be set for each patient by a user (for example, a doctor) who specifies use date data and is in charge of the patient, and transmits the personal calendar screen data to the client terminal 3.

A registration unit 11e receives the use date data from the client terminal 3 and registers therein the use date data.

When first liquid level data received from the delivery unit 17 becomes equal to or lower than a first threshold, which is a reference at the time of decrease, a transmission unit 11h assumes that there is an additional received-order of the coolant gas, and transmits an additional request of the coolant gas to the client terminal 3 on the deliverer side.

When second liquid level data received from the delivery unit 17 returns to a value equal to or higher than a second threshold, which is a reference at the time of replenishment, a replenishment-fee adding unit 11j assumes that there is replenishment of the coolant gas and adds a fee associated with the replenishment to the charging information and transmits the charging information to the client terminal 3 on the deliverer side.

According to the present embodiment, the received-order management server 11 can calculate the delivery date data and the collection date data based on the article code and the use date data received from the user terminal 3 on the user side, and select an optimum container type for delivering the article from the plural kinds of containers based on the article code. The received-order management server 11 can also calculate a billing amount by multiplying a unit price by a rental period of the delivery unit 17 from the delivery date to the collection date, by receiving a delivery completion notification representing that the delivery unit 17 has arrived at the user side and a collection completion notification representing that the collection operation has been completed by the collection operation to be performed on a collection date calculated at the time of placing an order.

The received-order management server 11 generates the personal calendar screen data so as to include a personal calendar region for specifying use date data in which the article code, personal identification data, delivery destination data of the article, and a date region are arranged, according to a request from a user, thereby enabling the user to register the use date data on the calendar screen.

According to the present embodiment, the received-order management server 11 can select an optimum container for delivering an article by using an article code as a key, by referring to a memory unit 11f in which plural kinds of article codes and plural kinds of container types are memorized in association with each other. Therefore, an optimum container according to the article can be selected easily and quickly.

The delivery date and the collection date can be accurately calculated by calculating the delivery date data as a date obtained by subtracting a delivery date lead time corresponding to delivery destination data of an article from the use date data received from the user terminal 3 on the user side, and calculating the collection date data as a date obtained by adding a collection date lead time corresponding to the delivery destination data of the article to the use date data which includes a date on which the article is to be used. Therefore, an accurate schedule can be notified to the user.

By calculating a billing amount based on a fixed unit price corresponding to the container type, a guideline at the time of selecting the container type becomes clear. Therefore, a user can know a total price for delivering the article accurately beforehand.

According to the present embodiment, since devices to be used in the container are selected according to the type of the article and a total value of unit prices corresponding to the used devices is set as a fixed unit price, the total value of unit prices can be calculated in detail for each article. Therefore, an accurate total value of unit prices corresponding to the article can be calculated.

Since the unit price is multiplied correspondingly to a monitored data amount during the rental period, a useless unit price corresponding to a period not being monitored can be excluded. Therefore, a unit price only for a period in which actual monitoring is being performed during the rental period can be acquired.

The delivery unit 17 can set the used state/unused state of each device from outside. And by setting the total value of unit prices of devices in the used state as the unit price, the received-order management server 11 can calculate the total value of unit prices in detail for each article. Therefore, an accurate total value of unit prices corresponding to the article can be calculated.

According to the present embodiment, since a discount rate is memorized for each orderer being a discount target, and the discount rate is acquired for each user code and is multiplied by the billing amount, detailed services can be performed to the user. Therefore, there is an effect of encouraging a user who has a high repeat rate to repeat the usage of the system.

The personal calendar screen that can be set for each patient by a user (for example, a doctor) who is in charge of the patient is displayed in which a plurality of date regions are arranged per month. Therefore, the user can specify the use date data on the calendar screen for each month, which enables to improve the user-friendliness for the user.

Since the used state/unused state of each device provided in the delivery unit 17 can be set from outside, the hardware configuration of the delivery unit 17 does not need to be changed. Therefore, the delivery unit 17 having the same configuration can be used as containers for various kinds of articles.

A threshold is set in the data of the thermometer Se1 in the delivery unit 17, and when the threshold reaches a critical value, an alarm can be generated. Therefore, a threshold can be not only individually set according to the type of the article or the importance of the article, but also it can be immediately recognized that the article has reached an abnormal state at the shipping address or the delivery destination.

A threshold is set in the data of the liquid level indicator Se2 in the delivery unit 17, and when the liquid level becomes equal to or lower than a first threshold, it is assumed that there is an additional received-order of the coolant gas, and an additional request is transmitted, and when the liquid level returns to a level equal to or higher than a second threshold, it is assumed that the coolant gas has been replenished, and the replenishment fee is charged. Therefore, excess and deficiency of the coolant gas can be automatically recognized, and the fee can be charged automatically.

### <Sequence diagram A>

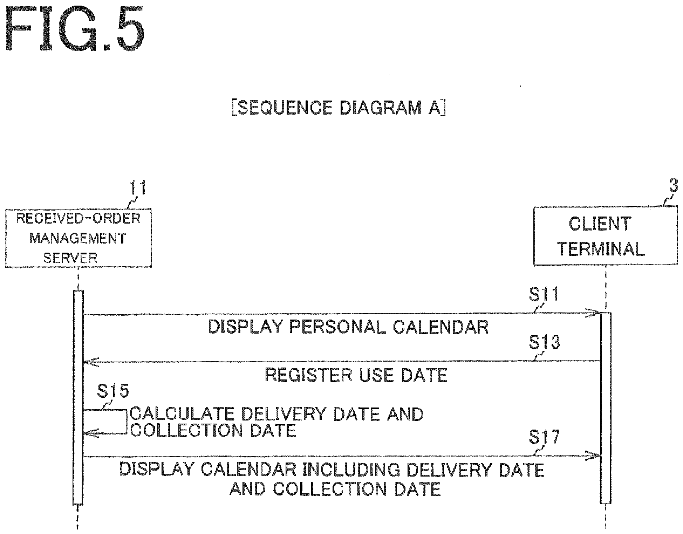

FIG. 5 is a sequence diagram of calendar screen generation according to the first embodiment of the present invention.

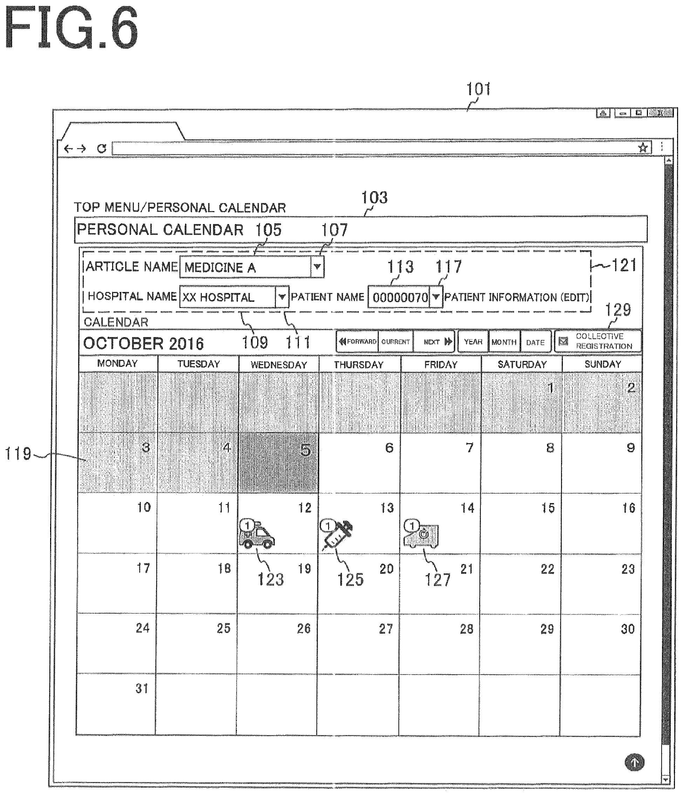

At Step S11, the received-order management server 11 transmits data displaying a personal calendar (see FIG. 6) to the client terminal 3.

At Step S13, the client terminal 3 displays the received personal calendar on a monitor, and registers a use date 125 selected by a user in the received-order management server 11. That is, the client terminal 3 transmits an article code and use date data to the received-order management server 11.

At Step S15, the received-order management server 11 calculates a delivery date 123 and a collection date 127 based on the use date 125. That is, the schedule calculation unit 11a calculates delivery date data associated with the delivery of an article, and collection date data associated with the collection of the delivery unit based on the article code and the use date data received from the client terminal 3 on the user side.

At Step S17, the received-order management server 11 transmits data displaying the delivery date 123 and the collection date 127 calculated by the received-order management server 11 to the client terminal 3.

### <Personal calendar screen in state of including use date>

FIG. 6 is a diagram illustrating a personal calendar screen in a state of including a use date by the client terminal according to the first embodiment of the present invention.

As a precondition for explanations, for example, it is assumed that an article name is "article A", a hospital name is "XX Hospital", and a patient name is "00000070". On an initial screen of the client terminal 3, a screen in which there is no mark data indicated by reference numerals 123, 125, and 127 is displayed, of the personal calendar 101 illustrated in FIG. 6.

In this example, there is a title 103 indicating that the screen is the personal calendar, and the "article A" is displayed in a frame 105 for the article name (a region specifying an article), and the contents in the frame 105 can be selected by using a pull-down button 107.

Below the frame 105, "XX hospital" is displayed in a frame 109 for the hospital name (a region specifying shipping address data), and the contents in the frame 109 can be selected by using a pull-down button 111. On the right thereof, "00000070" is displayed in a frame 113 for the patient name (a region specifying personal identification data), and the contents in the frame 113 can be selected by using a pull-down button 117. Further, a "collective registration" button 129 is provided on the right thereof, which is used in a case where scheduled dates for registration are input collectively.

Further, in a region on the lower side thereof, there is a calendar region 119. In this example, a calendar of October 2016 is displayed.

In this example, the delivery date 123 is displayed on October 12th, and the use date 125 is displayed on October 13th, and the collection date 127 is displayed on October 14th.

In this manner, the received-order management server 11 generates the personal calendar screen data so as to include the personal calendar region for specifying the use date data, in which the article code, the personal identification data, shipping address data of the article, and a date region are arranged, in response to a request from a user. Therefore, the user can register the use date data on the calendar screen.

Accordingly, the user can easily specify the use date of the storage container from the user terminal, thereby enabling to improve the user-friendliness.

### <ER diagram>

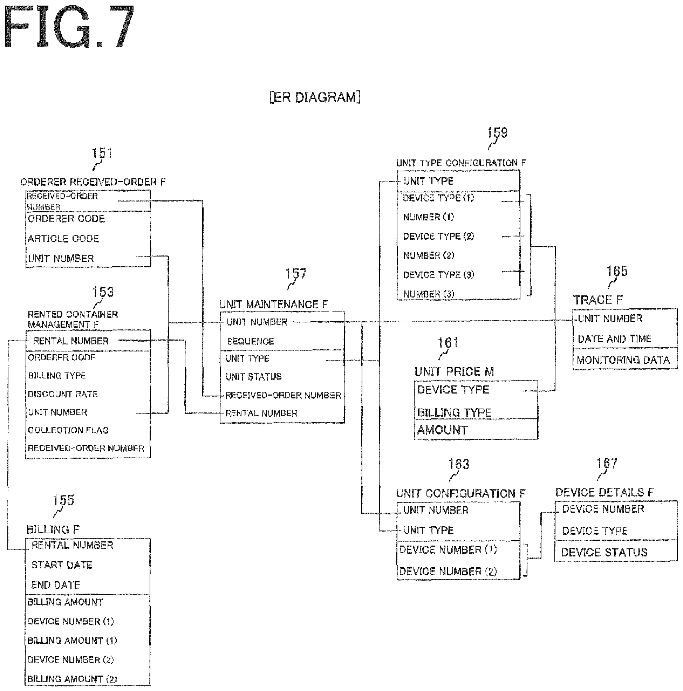

FIG. 7 is an ER diagram illustrating an association of files to be processed by the received-order management server according to the first embodiment of the present invention.

Upon reception of an orderer code and an article code from the client terminal 3, the received-order management server 11 memorizes the orderer code, the article code, and a unit number in an orderer received-order file 151 corresponding to a received-order number. The unit number is identified according to which unit is allocated to the received-order by the received-order management server 11.

The received-order management server 11 extracts the received-order number from the orderer received-order file 151 by using the unit number as a key, extracts a rental number from a rented container management file 153, and memorizes a unit type, a unit status, the received-order number, and the rental number in a unit maintenance file 157 corresponding to the unit number and the sequence.

The received-order management server 11 memorizes a device type (1), a number thereof (1), a device type (2), a number thereof (2), a device type (3), and a number thereof (3) in a unit type configuration file 159 corresponding to the unit type.

The received-order management server 11 memorizes a device number (1) and a device number (2) in a unit configuration file 163 corresponding to the unit number and the unit type.

The received-order management server 11 memorizes a device status in a device details file 167 corresponding to the device number and the device type.

The received-order management server 11 also memorizes a billing amount, a device number (1), a billing amount (1), a device number (2), and a billing amount (2) in a billing file 155 corresponding to the rental number, start date, and end date.

The received-order management server 11 memorizes orderer code, billing type, discount rate, unit number, collection flag, and received-order number in the rented container management file 153 corresponding to the rental number.

The received-order management server 11 also memorizes the amount in a unit price master 161 corresponding to the device type and the billing type.

The received-order management server 11 memorizes monitoring data in a trace file 165 corresponding to the unit number and date and time, by update processing as required.

Similarly, the received-order management server 11 memorizes a device status in the device details file 167 corresponding to the device number and the device type by the update processing as required.

### <Work sequence diagram>

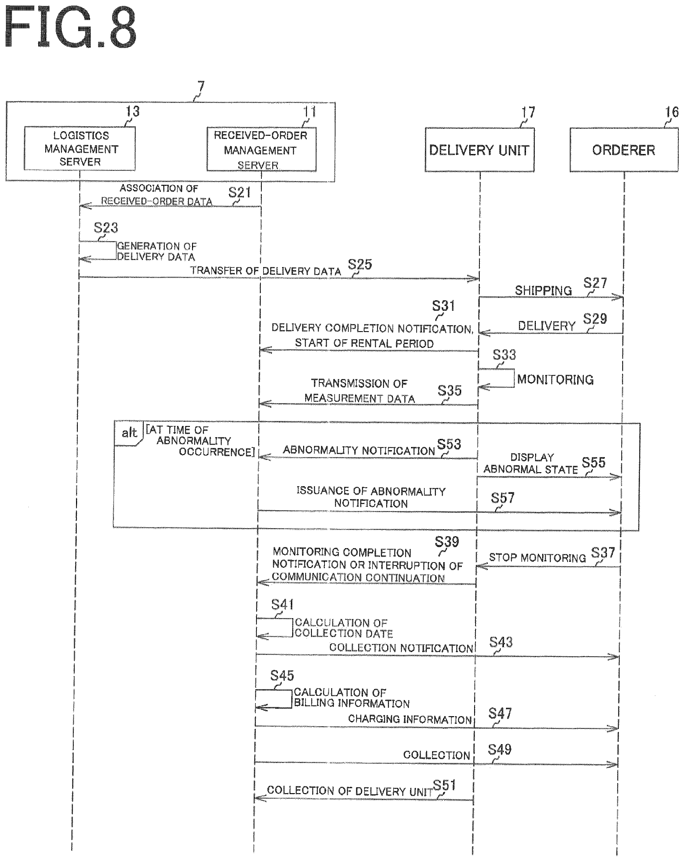

FIG. 8 is a work sequence diagram illustrating a container rental procedure according to the first embodiment of the present invention.

In the present embodiment, a flow in the order of new shipping, delivery, and collection is described.

At Step S21, the received-order management server 11 performs association of received-order data (including the article code), and transmits the result thereof to the logistics management server 13. Here, association of received-order data represents generation of delivery data.

The selection unit 11b of the received-order management server 11 selects an optimum container type for delivering the article from the plural kinds of containers based on the article code.

At Step S23, the received-order management server 11 generates delivery data based on the association of the received-order data.

At Step S25, the received-order management server 11 transfers the delivery data to the delivery unit 17, and performs a packing operation (storing articles to be shipped in the delivery unit 17) on the delivery date.

At Step S27, the delivery unit 17 sends an output itemized invoice to the orderer 16.

At Step S29, the orderer 16 delivers the articles based on the itemized invoice.

At Step S31, the delivery unit 17 transmits a delivery completion status (start of a rental period) to the received-order management server 11, after completion of delivery.

At Step S33, the delivery unit 17 also performs a monitoring operation of the unit for maintaining the quality even during the rental period.

At Step S35, the delivery unit 17 transmits measurement data acquired by the monitoring operation to the received-order management server 11.

At Step S37, when judging that the monitoring operation may be finished, the orderer 16 instructs the delivery unit 17 to stop monitoring.

At Step S39, upon reception of the monitoring stop instruction, the delivery unit 17 transmits a monitoring completion notification or a status indicating that the communication has been interrupted to the received-order management server 11.

At Step S41, the received-order management server 11 calculates the collection date.

At Step S43, the received-order management server 11 transmits collection notification data regarding the calculated collection date to the orderer 16.

At Step S45, the received-order management server 11 calculates billing information based on the calculated collection date.

At Step S47, the received-order management server 11 generates charging information based on the calculated billing information and transmits the charging information to the orderer 16.

At Step S49, a deliverer visits the orderer on the collection date calculated at the time of placing the order to collect the rented container.

At Step S51, the vehicle 21 collects the delivery unit 17.

At the time of abnormality occurrence in the measurement value (in a square frame), at Step S53, the delivery unit 17 transmits an abnormality notification to the received-order management server 11.

At Step S55, the delivery unit 17 displays an abnormal state.

At Step S57, the received-order management server 11 issues an abnormality notification informing an abnormality content to the orderer 16.

In this manner, the received-order management server 11 calculates the delivery date data and the collection date data based on the article code and the use date data received from the user terminal 3 on the user side, and selects an optimum container type for delivering the article from the plural kinds of containers based on the article code. The received-order management server 11 then receives the delivery completion notification indicating that the delivery unit 17 has arrived at the user side and the collection completion notification indicating that the collection operation has been completed according to the collection operation to be performed on the collection date calculated at the time of placing the order, thereby enabling to calculate the billing amount by multiplying the unit price by the rental period of the delivery unit 17 from the delivery date to the collection date.

Therefore, an optimum container type can be selected according to the type of the article, to calculate the billing amount based on the rental period of the container accurately. Further, the rental fee of the container that can be used at the time of delivering the article while ensuring the quality, and can be used also at the time of storing the article at the delivery destination can be calculated.

### <Sequence diagram>

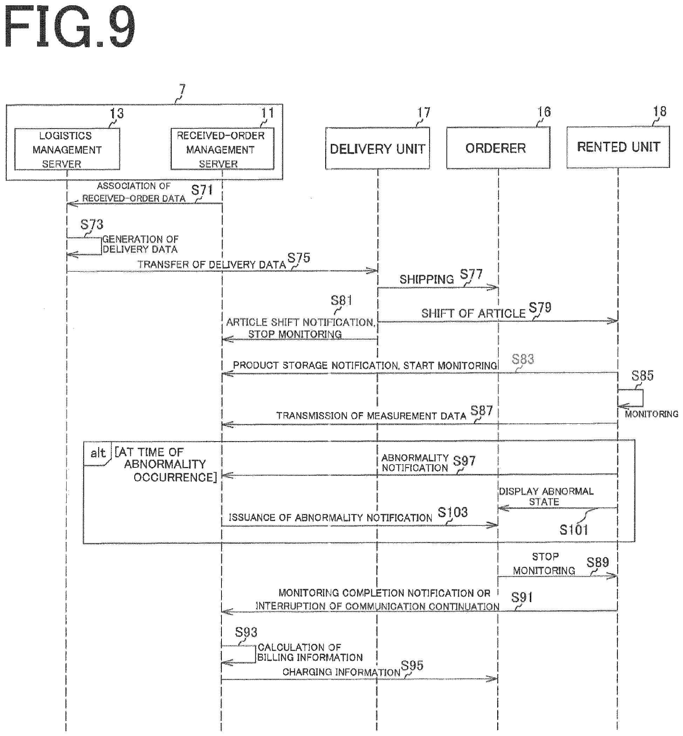

FIG. 9 is a sequence diagram illustrating a replenishment flow to a rented container according to the first embodiment of the present invention.

In the present embodiment, a flow in the order of additional shipment, delivery, and replacement of the container is described.

At Step S71, the received-order management server 11 performs association of received-order data and transmits the result thereof to the logistics management server 13.

At Step S73, the logistics management server 13 generates shipping data based on the association of the received-order data.

At Step S75, the logistics management server 13 transfers the shipping data to the delivery unit 17, and performs a packing operation (storing articles to be shipped in the delivery unit 17) on the delivery date.

At Step S77, the delivery unit 17 sends an output itemized invoice to the orderer 16.

At Step S79, the delivery unit 17 shifts the articles to a rented unit 18.

Here, the rented unit represents the delivery unit 17 that is rented to the orderer.

At Step S81, the delivery unit 17 transmits a notification indicating that the articles have been shifted and a request to stop monitoring to the received-order management server 11.

At Step S83, the rented unit 18 transmits an article storage notification and information to start monitoring of the article to the received-order management server 11.

At Step S85, the rented unit 18 starts monitoring of the articles.

At Step S89, when judging that the monitoring operation may be finished, the orderer 16 instructs the rented unit 18 to stop monitoring.

At Step S91, upon reception of the monitoring stop instruction, the rented unit 18 transmits a monitoring completion notification to the received-order management server 11 or interrupts continuation of communication.

At Step S93, the received-order management server 11 calculates billing information based on the calculated collection date.

At Step S95, the received-order management server 11 generates charging information based on the calculated billing information and transmits the charging information to the orderer 16.

When an error occurs in the measurement value (in a square frame), at Step S97, the rented unit 18 transmits an abnormality notification to the received-order management server 11.

At Step S101, the rented unit 18 displays an abnormal state to the orderer 16.

At Step 103, the received-order management server 11 issues an abnormality notification informing an abnormality content to the orderer 16.

In this manner, the received-order management server 11 calculates the delivery date data and the collection date data based on the article code and the use date data received from the user terminal 3 on the user side, and selects an optimum container type for delivering the article from plural kinds of containers based on the article code. Then, the received-order management server 11 can calculate a billing amount by multiplying a unit price by a rental period calculated based on a delivery completion notification indicating that the delivery unit 17 has arrived at a user side and a collection completion notification indicating that a collection operation has been completed according to the collection operation to be performed on the collection date calculated at the time of placing an order, which are received from the delivery unit 17.

Therefore, an optimum container type can be selected according to the type of the article, to calculate the billing amount based on the rental period of the container accurately. Further, the rental fee of the container that can be used at the time of delivering the article while ensuring the quality, and can be used also at the time of storing the article at the delivery destination can be calculated.

### <Flowchart>

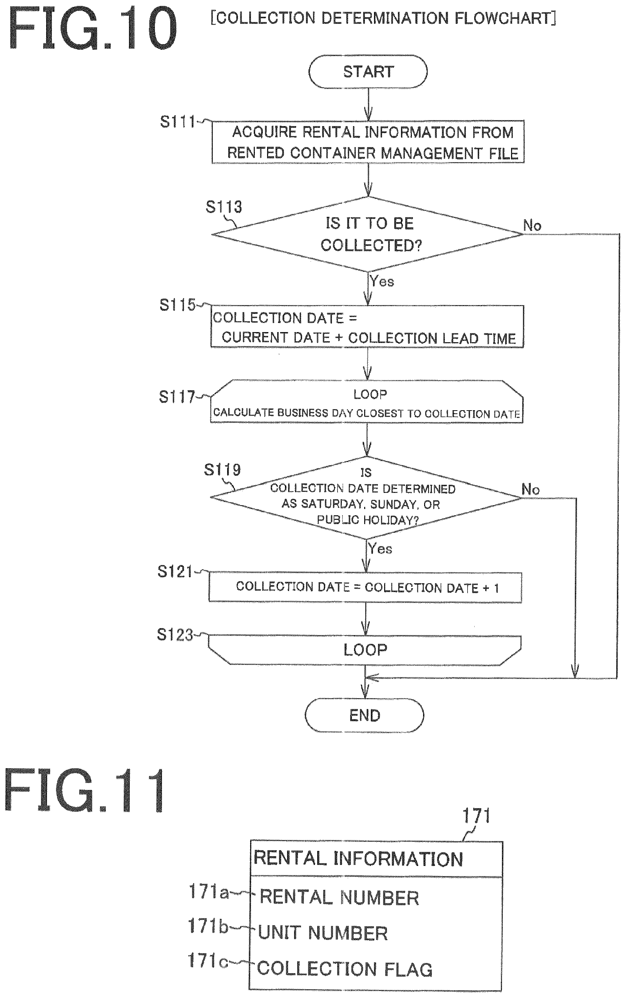

FIG. 10 is a flowchart of collection determination according to the first embodiment of the present invention.

At Step S111, the received-order management server 11 acquires rental information (rental number, unit number, and collection flag, see FIG. 11) illustrated in FIG. 11 from the rented container management file 153.

At Step S113, the received-order management server 11 judges whether a rented container is a collection target. That is, when the collection flag included in the rental information is "0", which indicates that the rented container is not a collection target, the received-order management server 11 ends the process. When the collection flag is "1", which indicates that the rented container is a collection target, the process proceeds to Step S115.

At Step S115, the schedule calculation unit 11a of the received-order management server 11 performs calculation of "collection date" = "current date" + "collection date lead time", in order to calculate the collection date.

At Step S117, the schedule calculation unit 11a calculates a business day closest to the collection date as a loop.

At Step S119, the schedule calculation unit 11a performs determination whether the collection date is Saturday, Sunday, or a public holiday. When the collection date is a "weekday", the schedule calculation unit 11a ends the process. On the other hand, when the collection date is Saturday, Sunday, or a public holiday, the process proceeds to Step S121.

At Step S121, the schedule calculation unit 11a performs calculation of "collection date" = "collection date" + 1, and repeats the process until the loop at Step S123 ends.

### <Configuration of rental information>

FIG. 11 is a diagram illustrating a configuration of rental information in a flowchart of collection determination according to the first embodiment of the present invention.

Rental information 171 is configured by a rental number 171a, a unit number 171b, and a collection flag 171c.

### <Flowchart>

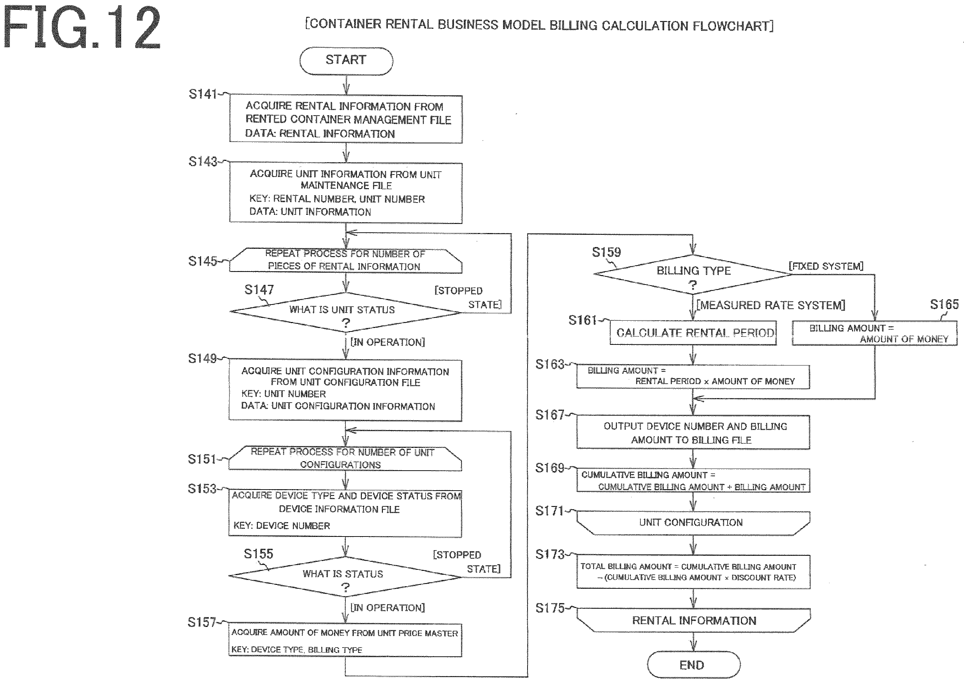

FIG. 12 is a flowchart of billing calculation according to the first embodiment of the present invention.

At Step S141, the billing-amount calculation unit 11c acquires rental information 171 from the rented container management file 153.

At Step S143, the billing-amount calculation unit 11c acquires unit information from the unit maintenance file 157. That is, the billing-amount calculation unit 11c extracts unit information from the unit maintenance file 157 by using the rental number and the unit number as a key.

At Step S145, the billing-amount calculation unit 11c repeats the calculation for the number of pieces of rental information.

At Step S147, when the unit status indicates a stopped state, the process proceeds to Step S145 and the process at Step S145 is repeated. On the other hand, when the unit status indicates "in operation", the process proceeds to Step S149.

At Step S149, the billing-amount calculation unit 11c acquires unit configuration information from the unit type configuration file 159. That is, the billing-amount calculation unit 11c extracts the unit configuration information from the unit type configuration file 159 by using the unit number as a key.

At Step S151, the billing-amount calculation unit 11c repeats the calculation for the number of unit configurations.

At Step S153, the billing-amount calculation unit 11c acquires the device type and the device status from the device details file 167. That is, the billing-amount calculation unit 11c acquires the device type and the device status from the device details file 167 by using the device number as a key.

At Step S155, when the status indicates a stopped state, the process proceeds to Step S151 and the process at Step S151 is repeated, and when the status indicates "in operation", the process proceeds to Step S157.

At Step S157, the billing-amount calculation unit 11c acquires an amount from the unit price master 161 by using the device type and the billing type as a key.

At Step S159, the billing-amount calculation unit 11c judges whether the billing type is set to a "measured rate system" in which billing corresponds to a rental period, or a "fixed system" of a certain amount.

At Step S161, a case where the billing type is the "measured rate system" is assumed, and the billing-amount calculation unit 11c calculates the rental period.

At Step S163, the billing-amount calculation unit 11c calculates the billing amount as "billing amount = rental period × amount of money".

On the other hand, at Step S165, a case where the billing type is the "fixed system" is assumed, and the billing-amount calculation unit 11c calculates the billing amount as "billing amount = amount of money".

At Step S167, the billing-amount calculation unit 11c outputs the billing amount calculated at either Step S163 or S165 and the device number to the billing file 155.

At Step S169, the billing-amount calculation unit 11c calculates a cumulative billing amount as "cumulative billing amount = cumulative billing amount + billing amount".

At Step S171, the billing-amount calculation unit 11c repeats the calculation for the number of unit configurations to calculate the billing amount.

At Step S173, the billing-amount calculation unit 11c calculates the total billing amount as "total billing amount = cumulative billing amount - (cumulative billing amount × discount rate)".

At Step S175, the billing-amount calculation unit 11c outputs the calculation result as the rental information.

### <Unit configuration information>

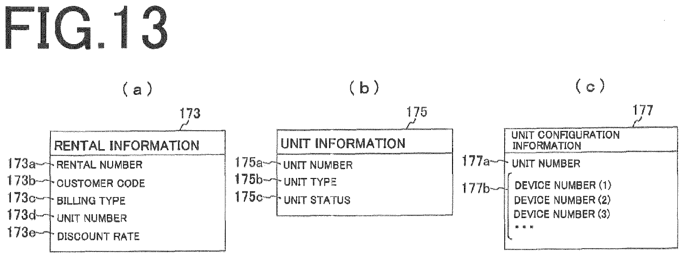

FIGS. 13 are diagrams illustrating configurations of (a) rental information, (b) unit information, and (c) unit configuration information, which are referred to in the flowchart of billing calculation according to the first embodiment of the present invention illustrated in FIG. 12.

As illustrated in FIG. 13(a), the rental information 173 is configured by a rental number 173a, an orderer code 173b, a billing type 173c, a unit number 173d, and a discount rate 173e.

As illustrated in FIG. 13(b), the unit information 175 is configured by a unit number 175a, a unit type 175b, and a unit status 175c.

As illustrated in FIG. 13(c), the unit configuration information 177 is configured by a unit number 177a, and device numbers (1) to (n) 177b.

### <Total amount for each unit type>

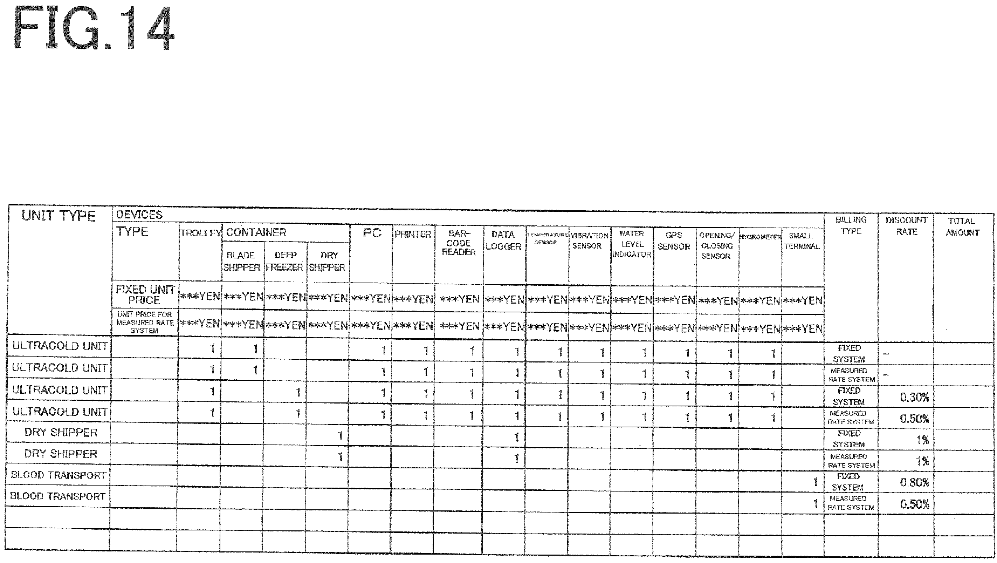

FIG. 14 is a diagram illustrating a total amount for each unit type according to the first embodiment of the present invention.

As illustrated in FIG. 14, unit type, devices, billing type, discount rate, and total amount are arranged horizontally. As a breakdown of devices, type, trolley, capacity, PC, printer, barcode reader, data logger, temperature sensor, vibration sensor, liquid level indicator, GPS sensor, opening/closing sensor, hygrometer, and small terminal are arranged. As the device type, there are fixed unit price and unit price for the measured rate system. Further, the container includes blade shipper, deep freezer, and dry shipper.

The unit type includes ultracold unit, dry shipper, and blood transport type. The billing type includes the fixed system and the measured rate system.

As illustrated in FIG. 14, the billing-amount calculation unit 11c calculates the total amount of required devices for each unit type. At this time, the billing type for each device is calculated, to calculate the total amount by subtracting the discount rate.

As the billing type, since there are the fixed system and the measured rate system, the billing type can be changed for each orderer.

The discount rate can be set for each orderer.

In this manner, the optimum container type can be selected according to the type of the article, and the billing amount can be accurately calculated based on the rental period of the container. Further, the rental fee of the container that can be used at the time of delivering the article while ensuring the quality thereof, and can be used at the time of storing the article at the delivery destination can be calculated.

### <Monitoring/status display screen>

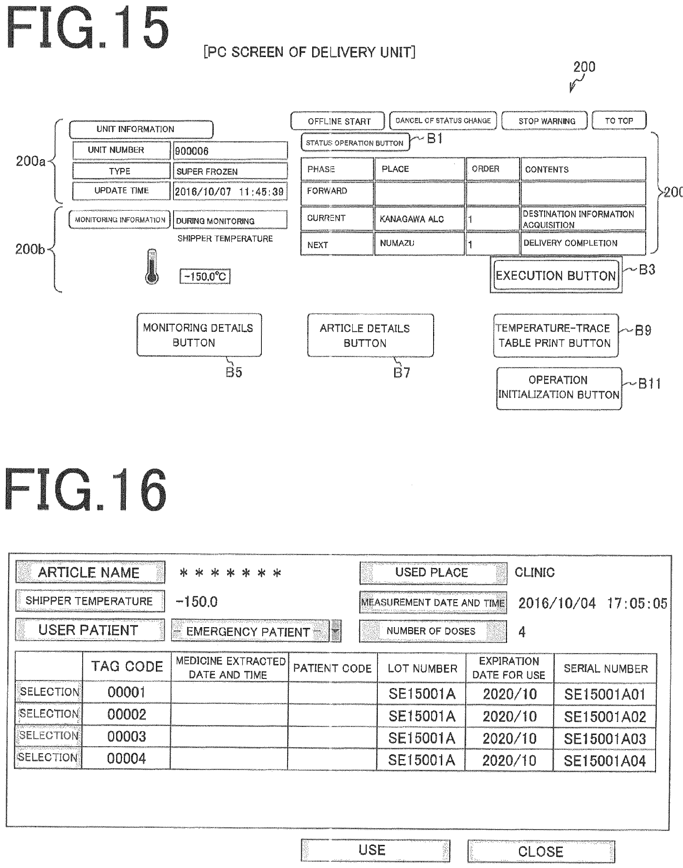

FIG. 15 is a diagram illustrating a monitoring/status display screen displayed on the PC in the delivery unit according to the first embodiment of the present invention.

On a monitoring/status display screen 200, unit number, type, and update time are displayed as unit information 200a, and temperature in the storage container 25 (shipper temperature) 200b is displayed as the monitoring information.

Further, on the monitoring/status display screen 200, a status operation button B1, phase, place, order, and contents are displayed and an execution button B3 is also displayed. When the status operation button B1 is pressed, the place, the order, and the work contents can be changed, and when the execution button B3 is pressed, the latest place, order, and work contents are transmitted from the PC 29 in the delivery unit 17 to the received-order management server 11.

Further, below the monitoring/status display screen 200, a monitoring details button B5, an article details button B7, a temperature-trace table print button B9, and an operation initialization button B11 are displayed.

### <Operation screen>

FIG. 16 is a diagram illustrating an operation screen to be used at the time of finishing monitoring of the delivery unit according to the first embodiment of the present invention.

As illustrated in FIG. 16, article name, used place, shipper temperature, measurement date and time, user patient, and number of uses are displayed on the operation screen, and tag code, extracted date and time, user patient, lot, expiration date for use, and serial number are further displayed.

When all the articles stored in the container in the delivery unit are used for patients, it becomes a state in which there is no article in the container, which means that monitoring is not required.

At this time, the status of the delivery unit becomes unused, and monitoring is stopped.

### <Second embodiment>

### <Modification of delivery unit>

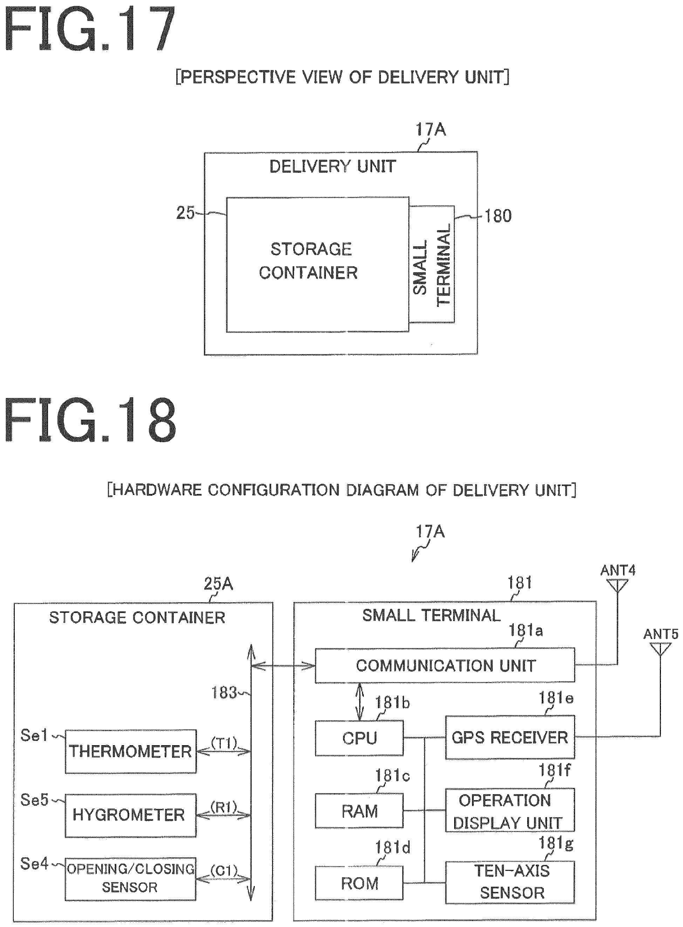

FIG. 17 is a perspective view illustrating a modification of the delivery unit according to a second embodiment of the present invention.

In the first embodiment, the delivery unit 17 illustrated in FIG. 3 is used as the delivery unit. In the second embodiment, a delivery unit 17A illustrated in FIG. 18 is used as the delivery unit.

The delivery unit 17A as a modification includes a storage container 25A, and a small terminal 180 that performs transmission and reception of information with the received-order management server 11 via the communication network 15.

The delivery unit 17A has a form in which the configuration of the delivery unit 17 illustrated in FIG. 2 is simplified, and a PC function, a GPS receiver 181e, and a ten-axis sensor 181g are provided in the small terminal 181.

### <Hardware configuration of modification of delivery unit>

FIG. 18 is a diagram illustrating a hardware configuration of the modification of the delivery unit according to the second embodiment of the present invention.

The storage container 25A can accommodate a plurality of articles therein, and includes the various sensors Se1, Se4, and Se5 attached therein or attached to outside thereof, and is connected with the small terminal 181 via a bus 183.

The thermometer Se1 is provided in the storage container 25A, to measure the ambient temperature in the storage container 25A, and outputs temperature data (T1) to a communication unit 181a.

The opening/closing sensor Se4 is provided in the storage container 25A, to detect whether a lid of the storage container 25A is in an opened state or in a closed state, and outputs the opening/closing data (C1) to the communication unit 181a.

The hygrometer Se5 is provided in the storage container 25A, to measure the ambient humidity in the storage container 25A, and outputs the humidity data (R1) to the communication unit 181a.

### The small terminal 181 includes the communication unit 181a, a CPU 181b, a RAM 181c, a ROM 181d, the GPS receiver 181e, an operation display unit 181f, and the ten-axis sensor 181g.

The communication unit 181a has an ANT 4, to transmit and receive data to and from the received-order management server 11 via the communication network 15.

The CPU 181b controls the entire operation of the delivery unit 17A according to a program memorized beforehand in the ROM 181d, by using the RAM 181c as a work memory.

The RAM 181c is a volatile memory medium capable of high speed reading and writing of information and can be used as a work memory.

The ROM 181d is a read-only non-volatile memory medium, and stores therein firmware and various kinds of data.

The GPS receiver 181e receives a radio signal from a plurality of GPS satellites via an antenna ANT 5 to calculate location information of the delivery unit 17A, and transmits the calculated location information to the received-order management server 11 via the communication network 15.

The operation display unit 181f includes a screen for displaying a menu for performing various setting and mode selection and key buttons, and receives various kinds of operation requests from a user.

The ten-axis sensor 181g is an inertial measurement unit corresponding to ten-axis measurement, and is a compound sensor in which a three-axis angular speed sensor (a gyroscope sensor), a three-axis acceleration sensor, a three-axis magnetic sensor, and a pressure sensor are sealed in one package, and is arranged in the small terminal so as to come in contact with a side face of the storage container 25A.

### Reference Signs List

1 received-order management system, 3 client terminal, 5, 15 communication network, 7 received-order management device, 9 front-end server, 11 received-order management server, 13 logistics management server, 17 delivery unit, 21 vehicle, 23 tag, 25 storage container, 27 wireless router, 29 PC, 31 printer, 33 battery, 35 alarm device, 37 GPS receiver, 39 bar-code reader, Se1 thermometer, Se2 liquid level indicator, Se3 vibration indicator, Se4 opening/closing sensor, Se5 hygrometer, Se6 voltage detection sensor, Se7 voltage detection sensor

## Claims

1. A received-order management system (1) comprising a server (11) configured to generate received-order data, and a delivery unit (17) for delivering a container (25) configured to accommodate an article therein, and to manage delivery of the delivery unit (17) and collection of the delivery unit (17) by connecting the server (11) and the delivery unit (17) with each other via a communication network (5, 15), wherein
the delivery unit (17) is configured to be able to mount therein a specific type of container (25) from plural kinds of containers (25) prepared according to a type of an article,
the delivery unit (17) includes
a liquid level indicator (Se2) configured to measure liquid level data associated with a liquid level of a coolant gas filled in a container (25) that stores an article therein,
the delivery unit (17) includes a plurality of sensors , and is able to set a used state or a unused state of the respective sensors depending on type of the article inside the delivery unit (17), and
a transmission unit (41) configured to transmit the liquid level data to the server (11).

## Patentansprüche

1. Ein Auftragseingangsverwaltungssystem (1), das einen Server (11), der dazu konfiguriert ist, Auftragseingangsdaten zu erzeugen, und eine Liefereinheit (17) zur Lieferung eines Behälters (25) aufweist, der dahingehend konfiguriert ist, einen Artikel darin aufzunehmen, und wobei ein Server eine Lieferung der Liefereinheit (17) und eine Abholung der Liefereinheit (17) durch Verbindung des Servers (11) und der Liefereinheit (17) miteinander über ein Kommunikationsnetzwerk (5, 15) verwaltet, wobei
die Liefereinheit (17) dahingehend konfiguriert ist, in derselben einen bestimmten Typ von Behälter (25) aus einer Mehrzahl von Arten von Behältern (25) befestigen zu können, die gemäß einem Artikeltyp vorbereitet sind,
die Liefereinheit (17) folgende Merkmale umfasst:
einen Flüssigkeitspegelanzeiger (Se2), der dazu konfiguriert ist, Flüssigkeitspegeldaten zu messen, die einem Flüssigkeitspegel eines Kühlmittelgases zugeordnet sind, das in einen Behälter (25) gefüllt wird, der einen Artikel darin lagert,
die Liefereinheit (17) eine Mehrzahl von Sensoren umfasst und in der Lage ist, einen Verwendungszustand oder einen Nicht-Verwendungszustand der jeweiligen Sensoren in Abhängigkeit vom Artikeltyp innerhalb der Liefereinheit (17) einzustellen, und
eine Sendeeinheit (41), die dazu konfiguriert ist, die Flüssigkeitspegeldaten an den Server (11) zu senden.

## Revendications

1. Système de gestion de commande reçue (1) comprenant un serveur (11) configuré pour générer des données de commande reçue, et une unité de livraison (17) pour livrer un récipient (25) configuré pour recevoir un article à l'intérieur, et pour gérer une livraison de l'unité de livraison (17) et une collecte de l'unité de livraison (17) en connectant le serveur (11) et l'unité de livraison (17) l'un à l'autre via un réseau de communication (5, 15), dans lequel
l'unité de livraison (17) est configurée pour pouvoir monter dans celle-ci un type spécifique de récipient (25) parmi plusieurs types de récipients (25) préparés en fonction d'un type d'un article,
l'unité de livraison (17) comprend
un indicateur de niveau de liquide (Se2) configuré pour mesurer les données de niveau de liquide associées au niveau de liquide d'un gaz réfrigérant rempli dans un récipient (25) qui stocke un article à l'intérieur,
l'unité de livraison (17) comprend une pluralité de capteurs et est en mesure de définir un état utilisé ou un état inutilisé des capteurs respectifs en fonction du type d'article à l'intérieur de l'unité de livraison (17), et
une unité de livraison (41) configurée pour transmettre les données relatives au niveau de liquide au serveur (11).
